# EUROPEAN PATENT SPECIFICATION

(11) **EP 3 072 551 B1**
(45) Date of publication and mention of the grant of the patent: **24.07.2019**
(21) Application number: 16154293.1
(22) Date of filing: 04.02.2016
(51) Int. Cl.: A61M 25/01, A61M 25/10, A61M 25/00, A61B 90/00

(54) **BALLOON CATHETER**
BALLONKATHETER
CATHETER A BALLONNET

(30) Priority: 27.03.2015 JP 2015065856
(43) Date of publication of application: 28.09.2016
(60) Divisional application: 19179666.3
(73) Proprietor: Asahi Intecc Co., Ltd., Seto-shi, Aichi 489-0071 (JP)
(72) Inventor: KATSURADA, Takeharu, Seto-Shi, Aichi 489-0071 (JP); MOGI, Takeshi, Seto-Shi, Aichi 489-0071 (JP)
(74) Representative: Winter, Brandl, Fürniss, Hübner, Röss, Kaiser, Polte - Partnerschaft mbB

(56) References cited:
- EP-A1- 1 721 631
- EP-A1- 2 389 973
- EP-A1- 2 719 418
- EP-A1- 2 762 192

## Description

### TECHNICAL FIELD

The present invention relates to a balloon catheter to be inserted into a stenosis site inside a blood vessel for expanding the stenosis site.

### BACKGROUND ART

Balloon catheters to be inserted into a stenosis site inside a blood vessel for expanding the stenosis site have already been proposed. A balloon catheter mainly comprises a balloon as an expanding body, an outer shaft joined to the proximal end of the balloon and an inner shaft inserted between the balloon and the outer shaft. Further, a metal marker member having radiopacity is commonly provided inside the balloon so that the position of the balloon can be detected under exposure of irradiation.

For example, Japanese Patent No. 3659664 discloses a catheter for expanding a blood vessel in which a platinum contrast marker (a marker member) is attached to a medical tube (an inner shaft) comprising an inner layer, a coil layer as a reinforcement layer and an outer layer.

Further, for example, Japanese Patent No. 3219968 discloses a balloon-expandable catheter in which a radiopaque marker fit in the inside of a depressed groove arranged on the outer periphery of an inner tube (an inner shaft) present in the inside of the balloon is covered by a heat-shrinkable tube thereover. Moreover, also disclosed is a balloon-expandable catheter in which a radiopaque marker arranged on the outer periphery of an inner pipe (an inner shaft) without providing a depressed groove is covered by a heat-shrinkable tube thereover.

EP 2389973 A1 discloses a balloon-expandable catheter comprising an inner shaft having an inner layer and a coil body arranged on the outer periphery of the inner layer and a marker arranged on the outer periphery of the coil body.

EP 1721631 A1 discloses a catheter comprising an inner layer, reinforcement layer arranged at the outer periphery of the inner layer and a marker arranged between the reinforcement layer and the inner layer.

EP 2719418 A1 discloses a balloon-expandable catheter comprising an inside tube having an inner layer, a support body arranged at the outer periphery of the inner layer and a marker arranged between the support body and the inner layer.

EP 2762192 A1 discloses a balloon catheter comprising an inner shaft having an inner layer, a braid and an outer layer.

### SUMMARY OF THE INVENTION

### TECHNICAL PROBLEM

However, the catheter for expanding a blood vessel disclosed in Japanese Patent No. 3659664 has the following disadvantages. Since a marker member is attached to the outer periphery of an inner shaft and exposed to the side of the inner surface of the balloon, there is a risk that the end portion of the marker member may damage the balloon. Further, a difference in rigidity may arise between a flexible inner shaft and a highly rigid marker member, creating a risk that the inner shaft is buckled due to an excessively concentrated stress at the attachment position of the marker member.

Moreover, in the balloon-expandable catheter disclosed in Japanese Patent No. 3219968 above, processing steps such as polishing and etching for providing a depressed groove. Therefore, the manufacturing process will be disadvantageously complicated. Further, disadvantageously, a site where the depressed groove is provided tends to have decreased strength due to a defective cross section. Furthermore, since the outer diameter of a balloon portion will be larger for a configuration in which a radiopaque marker arranged on the outer periphery of an inner tube (an inner shaft) is covered by a heat-shrinkable tube, the diameter of the balloon catheter is difficult to be reduced. Further, disadvantageously, in a case where a marker member is made of a highly rigid metal, the marker member itself is difficult to be deformed to follow the curvature of a blood vessel and the like, and it may easily be stuck at a position where the marker member is arranged.

Accordingly, an object of the present invention is to provide a balloon catheter in which there is no risk of damaging a balloon, and the diameter of a portion where a marker member is arranged can be reduced, and struck state at a position where the marker member is arranged can be prevented even when inserted into a curved blood vessel.

### SOLUTION TO PROBLEM

The present invention is characterized by a balloon catheter comprising a balloon, an outer shaft joined to the balloon and an inner shaft joined to the balloon, the inner shaft comprising an inner layer, a coil body arranged at the outer periphery of the inner layer and having a sparsely wound portion where gaps are formed between the adjacent turns of a wire, a marker member arranged at the outer periphery of the coil body, and an outer layer covering the coil body and the marker member, wherein at least one of the distal end part and the proximal end part of the marker member is arranged at a position facing a void formed between adjacent turns of the wire of the sparsely wound portion, wherein the at least one of the distal end part and the proximal end part of the marker member is curved toward the inner layer. In the present invention, the coil body has a sparsely wound portion where a wire is sparsely wound. I.e., the coil has a plurality of turns formed by the wire.

When the above balloon catheter in this configuration is inserted into a curved blood vessel, at least one of the distal end part and the proximal end part of the marker member can enter into the void formed between adjacent turns of the wire of the coil body. Therefore, stress concentration generated at a location where the marker member is arranged can be relieved, preventing the inner shaft from buckling. Further, the end portion of the marker member can be prevented from projecting toward the side of the inner surface of the balloon. Moreover, the manufacturing process will not be complicated because the marker member is covered by the outer layer without using another member. Furthermore, there is no risk that the marker would member damage the balloon. In addition, an excessively enlarged outer diameter of the inner shaft in the inside of the balloon can be prevented because the outer layer covering the coil body in this way also serves to cover the marker member.

Note that for example, in a case where the distal end part of the marker member is arranged at a position facing a void, at least a portion of the distal end part may be arranged in a position facing the void. Similarly, in a case where the proximal end part of the marker member is arranged at a position facing a void, at least a portion of the proximal end part may be arranged in a position facing the void.

Further, at least one of the distal end part and the proximal end part may form a smooth and continuous tapered shape. Therefore, rupture (burst or breakage) of the balloon due to the marker member can be prevented. Further, even in a case where external force is applied to the marker member when the balloon catheter curves, an edge of the marker member entered into the void can make contact with the wire of the coil body, allowing the movement of the marker member to be controlled.

Further, the marker member may be formed by a parallelogram metal plate wound on the outer periphery of the coil body so that the distal end part and the proximal end part are arranged at a position facing a void. Preferably, the parallelogram metal plate may have an inner angle corresponding to a pitch angle of wound inclination of the coil body.

### ADVANTAGEOUS EFFECT OF THE INVENTION

According to the balloon catheter of the present invention, there is no risk of damaging a balloon, and the diameter at a portion where a maker member is arranged can be reduced, and in addition, struck state at a position where the maker member is arranged can be prevented even when inserted into a curved blood vessel.

### BRIEF DESCRIPTION OF THE DRAWINGS

Fig. 1 shows a partial cross-sectional top view of a balloon catheter according to Example 1 (reference example).
Fig. 2 shows a partial enlarged cross-sectional view of the balloon catheter according to Example 1 (reference example).
Fig. 3 shows a partial enlarged cross-sectional view illustrating a state of the balloon catheter according to Example 1 (reference example) when curved.
Fig. 4 shows a partial enlarged cross-sectional view of the balloon catheter according to Example 2 (reference example).
Fig. 5 shows a partial enlarged cross-sectional view of the balloon catheter according to Example 3.
Fig. 6 shows a diagram illustrating a marker member of the balloon catheter according to Example 4.

### DESCRIPTION OF EMBODIMENTS

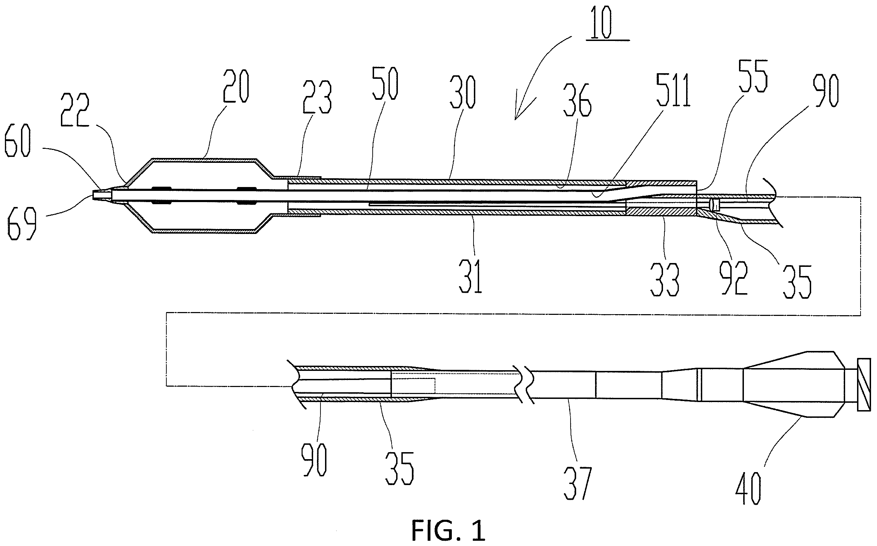

Below, Examples in which the balloon catheters will be described in detail. Note that in Figs. 1 to 5, the left side corresponds to the distal end side (the distal side) which is to be inserted into the body, and the right side corresponds to the proximal end side (the proximal side) which is to be operated by an operator such as a physician. Further, for easier understanding, those smaller than other parts such as a marker member 70 arranged inside a balloon 20 are shown in a somewhat exaggerated manner relative to the dimensions of other members.

### [Example 1] (reference example)

For example, a balloon catheter 10 used for treating a stenosis site inside a blood vessel of the heart and the like comprises a balloon 20, an outer shaft 30, a connector 40, an inner shaft 50 and a tip 60 as shown in Fig. 1.

The balloon 20 serves to expand a stenosis site, and comprises a member made of a resin. In addition, it has a distal end attachment part 22 in the distal end side and has a proximal end attachment part 23 in the proximal end side. The distal end attachment part 22 is joined to the distal end of the inner shaft 50 through the tip 60, and the proximal end attachment part 23 is joined to the distal end of the outer shaft 30.

The outer shaft 30 serves to feed a fluid, and comprises a tubular member which constitutes an inflation lumen 36 for feeding the fluid. Further, the outer shaft 30 has a distal end outer shaft portion 31, a guide wire port portion 33, a middle outer shaft portion 35 and a proximal end outer shaft portion 37 in the order from the distal end side. Note that the guide wire port portion 33 corresponds to a portion in which the distal end outer shaft portion 31, the middle outer shaft portion 35 and the inner shaft 50 are joined.

As further described in detail, the inner shaft 50 is inserted into the distal end outer shaft portion 31, and the inflation lumen 36 is formed between the distal end outer shaft portion 31 and the inner shaft 50. Moreover, the proximal end outer shaft portion 37 comprises a metal tubular member referred to as a so-called hypotube. In addition, the distal end of the proximal end outer shaft portion 37 is inserted into and joined to the proximal end of the middle outer shaft portion 35. Further, the connector 40 is attached to the proximal end of the proximal end outer shaft portion 37. Accordingly, when a liquid for expanding the balloon 20 such as a contrast agent and physiological saline is fed from an indeflator (not shown) attached to the connector 40, the liquid flows into the balloon 20 through the inflation lumen 36, allowing the balloon 20 to expand.

Note that the distal end outer shaft portion 31 and the middle outer shaft portion 35 are preferably configured with a tube comprising a resin such as polyamide, polyamide elastomer, polyolefine, polyester, or polyester elastomer. Further, the proximal end outer shaft portion 37 is preferably configured with a superelastic alloy such as stainless steel (SUS304) and a Ni-Ti alloy.

Moreover, the core wire 90 is attached to the inner surface of the distal end of the proximal end outer shaft portion 37. The core wire 90 has a circular cross-section, and configured with a tapered metal wire with a diameter decreased toward the distal end. In addition, the core wire 90 passes through the middle outer shaft portion 35 and the guide wire port portion 33, and is formed to extend through the distal end outer shaft portion 31. Further, the core wire 90 has a pressing member 92 near the proximal end of the guide wire port portion 33. By this, when a pushing force and a rotating force are applied to the core wire 90, the pressing member 92 makes contact with the guide wire port portion 33, allowing the pushing force and the rotating force to be transmitted to the distal end outer shaft portion 31 and the inner shaft 50. Alternatively, the core wire 90 may be allowed to make contact with the guide wire port portion 33 instead of providing the pressing member 92. By this, when a pushing force and a rotating force are applied to the core wire 90, the pushing force and the rotating force can be transmitted to the distal end outer shaft portion 31 and the inner shaft 50. Note that the core wire 90 is preferably configured with a superelastic alloy such as stainless steel (SUS304) and an Ni-Ti alloy.

Further, a proximal end side guide wire port 55 is formed at the proximal end of the inner shaft 50 by being joined to the guide wire port portion 33 of the outer shaft 30. Furthermore, a distal end side guide wire port 69 is formed at the tip 60 arranged at the distal end of the inner shaft 50.

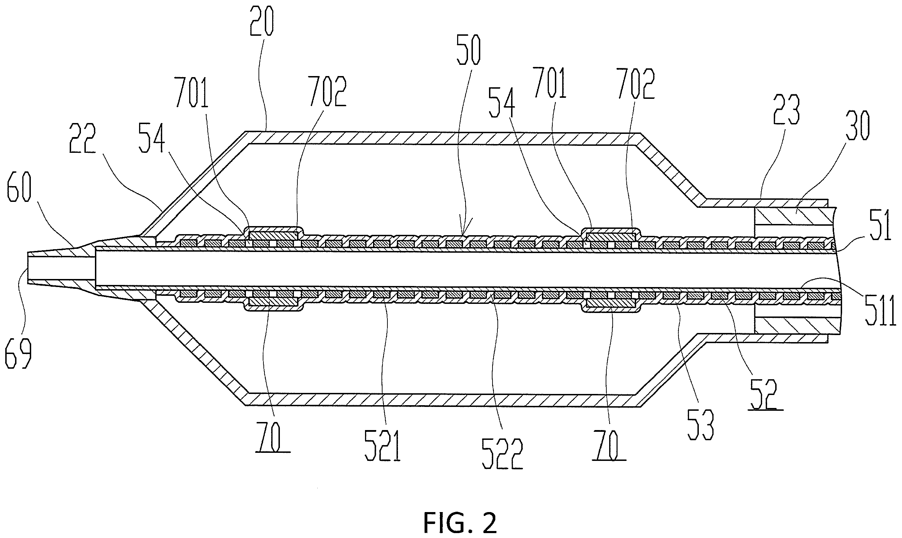

Moreover, as shown in Fig. 2, the inner shaft 50 has a tubular inner layer 51 in the inside thereof, the tubular inner layer 51 forming a guide wire lumen 511 for inserting a guide wire (not shown).

Moreover, a coil body 52 covering the inner layer 51 is arranged at the outer periphery of the inner layer 51. The coil body 52 has a sparsely wound portion 522 where a wire 521 with a rectangular cross-section is sparsely wound. I.e. the coil body 52 has a plurality of turns and gaps formed between adjacent wire 521, respectively.

Moreover, two of the marker members 70 with a cylindrical shape are arranged at each of the distal side and the proximal side on the outer periphery of the sparsely wound portion 522 of the coil body 52 in the inside of the balloon 20. Furthermore, an outer layer 53 covering the coil body 52 and the marker member 70 is formed.

Note that the inner layer 51 and the outer layer 53 are preferably configured with a resin such as polyethylene, polyurethane, polyamide, polyamide elastomer, polyolefine, polyester or polyester elastomer. Note that the coil body 52 is preferably configured with a superelastic alloy such as stainless steel (sus304) and a Ni-Ti alloy. Moreover, the marker member 70 is preferably configured with a radiopaque metal material such as platinum and tungsten.

Further, the gaps between the adjacent turns of the wire 521 are filled with the outer layer 53 at a region between the adjacent turns of the wire 521 of the coil body 52 where the marker member 70 is not arranged. In contrast, in a region where the marker member 70 is arranged, a void 54 without being filled with the outer layer 53 is formed. In addition, a distal end part 701 and a proximal end part 702 of the marker member 70 are arranged at a position facing the void 54.

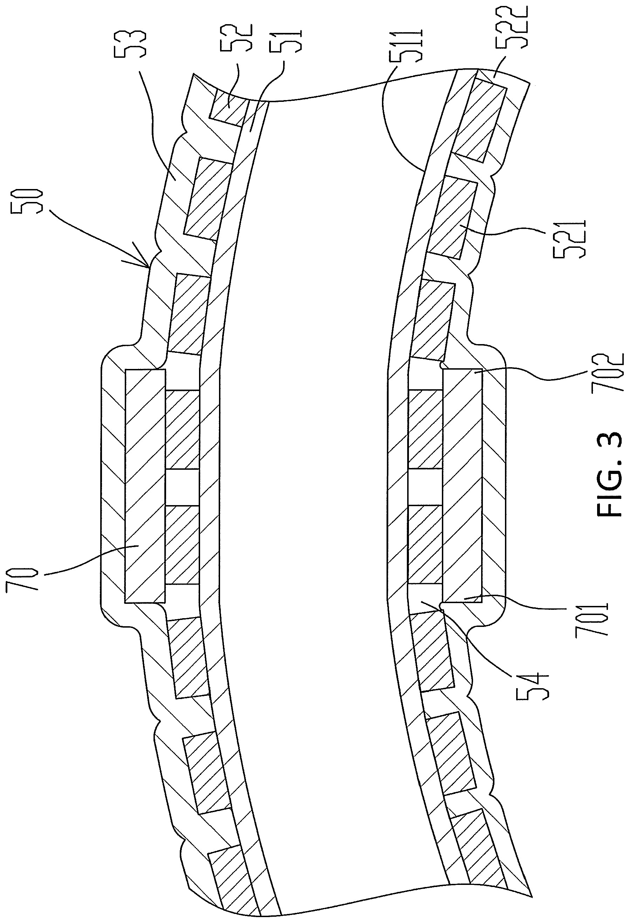

In the balloon catheter 10 having the above configuration, when the balloon catheter 10 is inserted into a curved blood vessel, the inner shaft 50 curves along with the blood vessel as shown in Fig. 3. At this time, a part of the distal end part 701 and a part of the proximal end part 702 of the marker member 70 can enter into the void 54 of the coil body 52 in the inside of the curved inner shaft 50. Therefore, stress concentration generated at a position where the marker member 70 is arranged can be relieved, preventing the inner shaft 50 from buckling. Further, the projection of the distal end part 701 and the proximal end part 702 of the marker member 70 toward the side of the inner surface of the balloon 20 can also be controlled. Furthermore, the marker member 70 can be covered by the outer layer 53 without using another member. Therefore, the manufacturing process will not be complicated, and there will be no risk of damaging the inner surface of the balloon 20. Moreover, the outer layer 53 also serves to cover the marker member 70 and the coil body 52. Therefore, the enlargement of the outer diameter of the inner shaft 50 inside the balloon 20 can be controlled, and the diameter of the inner shaft 50 can be reduced.

### [Example 2] (reference example)

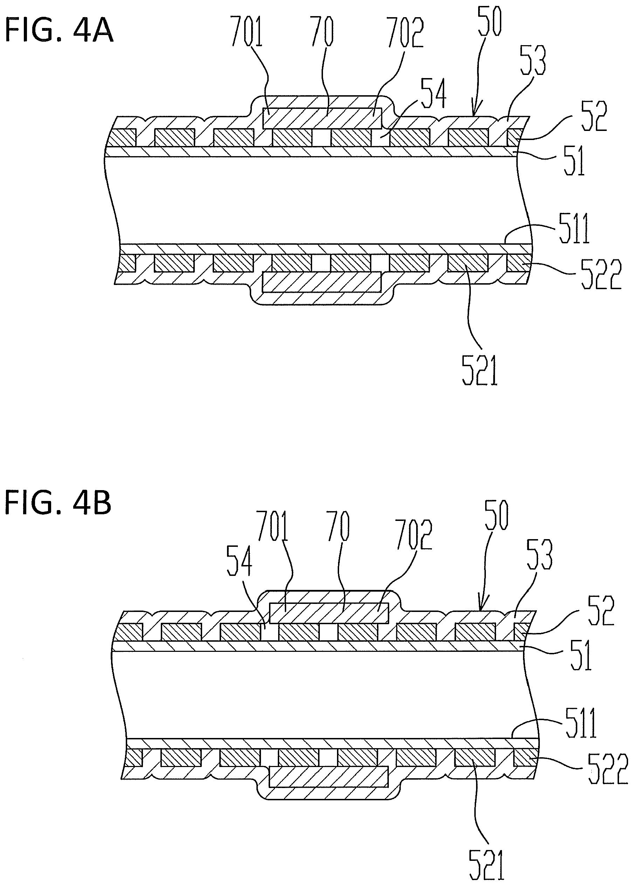

Below, Example 2 will be described with reference to Figs. 4A and 4B. Note that description is omitted for parts common with the above Example 1, and the same reference numbers are assigned in the figures.

Both the distal end part 701 and the proximal end part 702 of the marker member 70 are preferably arranged at a position facing the void part 54, but buckling of the inner shaft 50 can be prevented even in the case of the configuration shown in Example 2.

Specifically, only the proximal end part 702 of the marker member 70 may be arranged at a position facing the void 54 as shown in Fig. 4A. In this case, the gaps between adjacent turns of the wire 521 facing the distal end part 701 of the marker member 70 are filled with the outer layer 53.

Further, only the distal end part 701 of the marker member 70 may be arranged at a position facing the void part 54 as shown in Fig. 4B. In this case, the gaps between the adjacent turns of the wire 521 facing the proximal end part 702 of the marker member 70 are filled with the outer layer 53.

### [Example 3]

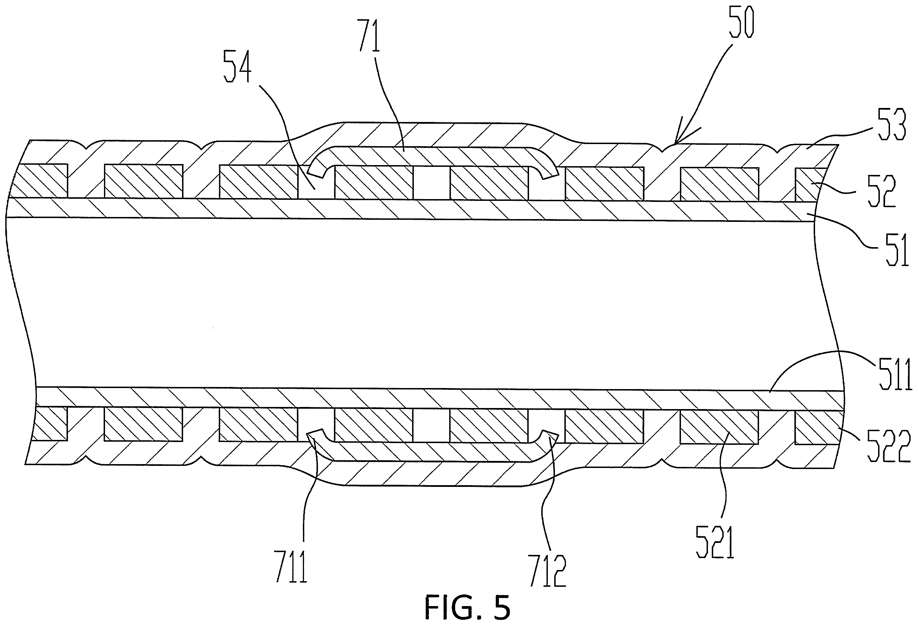

Below, Example 3 will be described with reference to Fig. 5. Note that description is omitted for parts common with the above Examples 1 and 2, and the same reference numbers are assigned in the figures.

As shown in Fig. 5, a distal end part 711 and a proximal end part 712 of a marker member 71 are preferably projected toward the void 54 by curving inside. In this configuration, the distal end part 711 and the proximal end part 712 form a smooth and continuous tapered shape. Therefore, a risk of damaging the inner surface of the balloon 20 with the end portion of the marker member 71 can be eliminated, and rupture (burst or breakage) of the balloon 20 due to the marker member 71 can be prevented. Further, even in a case where the balloon catheter 10 curves to apply external force to the marker member 71, the end portion of the marker member 71 which has entered into the void 54 can make contact with the turns of the wire 521 of the coil body 52 to control the movement of the marker member 71.

Note that a configuration may be such that at least one of the distal end part 711 and the proximal end part 712 is curved toward the inner layer 51 although a configuration is preferably such that the distal end part 711 and the proximal end part 712 of the marker member 71 are curved toward the inner layer 51.

### [Example 4]

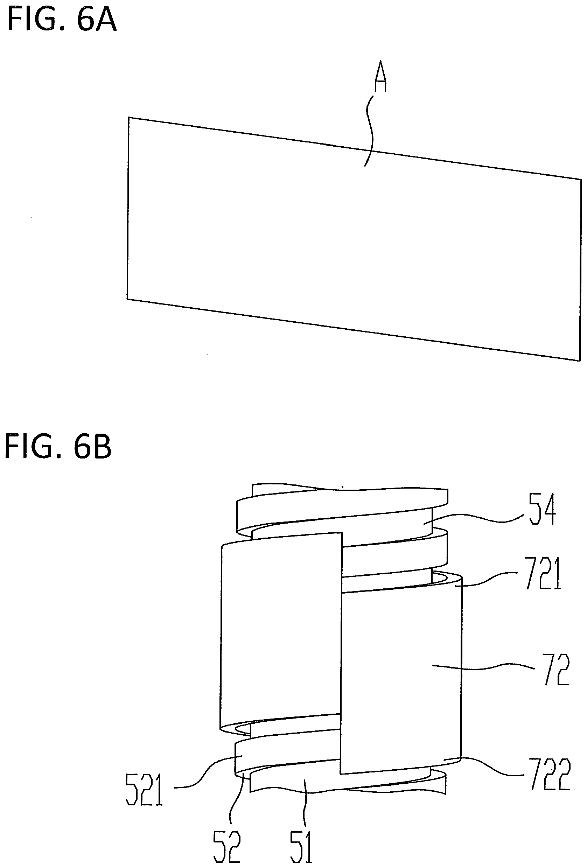

Below, Example 4 will be described with reference to Figs. 6A and 6B. Note that description is omitted for parts common with the above Examples 1 to 3, and the same reference numbers are assigned in the figures.

A marker member 72 may be formed by subjecting a parallelogram metal plate A in a plane view as shown in Fig. 6A to curving process. As shown in in Fig. 6B, the parallelogram metal plate A is wound on the outer periphery of the coil body 52 so that a distal end part 721 and a proximal end part 722 are continuously arranged at positions facing voids 54. Then, as shown in Fig. 6B, the distal end part 721 and the proximal end part 722 of the marker member 72 are almost entirely arranged at a position facing the void 54. Further, a configuration may be such that the distal end part 721 and the proximal end part 722 of the marker member 72 are curved toward the inner layer 51.

In Examples 1 to 4, the number of the marker members 70 to 72 to be provided inside the balloon 20 may be one or more. Moreover, the cross-sectional shape of the turns of the wire 521 of the coil body 52 shall not be limited to a rectangular cross-section, but may be of other shapes such as a circular cross-section.

### DESCRIPTION OF SYMBOLS

10 Balloon catheter
20 Balloon
30 Outer shaft
50 Inner shaft
51 Inner layer
52 Coil body
53 Outer layer
54 Void part
70, 71, 72 Marker member
521 Wire
522 Sparsely wound portion
701, 711, 721 Distal end part
702, 712, 722 Proximal end part

## Claims

1. A balloon catheter (10) comprising:
a balloon (20);
an outer shaft (30) joined to the balloon (20); and
an inner shaft (50) joined to the balloon (20), the inner shaft (50) comprising
an inner layer (51);
a coil body (52) arranged on the outer periphery of the inner layer (51) and having a plurality of turns of a wire (521), wherein the coil body (52) has a sparsely wound portion (522) where gaps are formed between the adjacent turns of the wire (521);
a marker member (70, 71, 72) arranged on the outer periphery of the coil body (52);
**characterized in that**
the balloon catheter (10) further comprises an outer layer (53) covering the coil body (52) and the marker member (71, 72),
at least one of a distal end part (711, 721) and a proximal end part (712, 722) of the marker member (71, 72) is arranged at a position facing a void (54) formed between adjacent turns of the wire (521) of the sparsely wound portion (522), and
the at least one of the distal end part (711, 721) and the proximal end part (712, 722) of the marker member (71, 72) is curved toward the inner layer (51).

2. The balloon catheter (10) according to claim 1, wherein at least one of the distal end part (711, 721) and the proximal end part (712, 722) of the marker member (71, 72) forms a smooth and continuous tapered shape.

3. The balloon catheter (10) according to claim 1 or 2, wherein both the distal end part (711, 721) and the proximal end part (712, 722) of the marker member (71, 72) are curved toward the inner layer (51).

4. The balloon catheter (10) according to claim 3, wherein the at least one of the distal end part (711, 721) and the proximal end part (712, 722) of the marker member (71, 72) resides completely inside the void (54).

5. The balloon catheter (10) according to any one of claims 1 to 4, wherein the marker member (72) is formed by a parallelogram metal plate (A) wound on the outer periphery of the coil body (52) so that the distal end part (721) and the proximal end part (722) are arranged at a position facing a void (54).

## Patentansprüche

1. Ballonkatheter (10) mit:
einem Ballon (20);
einem an den Ballon (20) angefügten Außenschaft (30); und
einem an den Ballon (20) angefügten Innenschaft (50), der aufweist
eine Innenschicht (51);
einen am Außenumfang der Innenschicht (51) angeordneten Wicklungskörper (52) mit einer Vielzahl von Windungen eines Drahts (521), wobei der Wicklungskörper (52) einen lose gewickelten Abschnitt (522) mit Lücken zwischen nebeneinanderliegenden Windungen des Drahts (521) hat; und
ein am Außenumfang des Wicklungskörpers (52) angeordnetes Markierungselement (70, 71, 72);
**dadurch gekennzeichnet, dass**
der Ballonkatheter (10) des Weiteren eine den Wicklungskörper (52) und das Markierungselement (71, 72) umhüllende Außenschicht (53) aufweist,
wenigstens einer aus einem distalen Endabschnitt (711, 721) und einem proximalen Endabschnitt (712, 722) des Markierungselements (71, 72) an einer Stelle gegenüber einer Lücke (54) zwischen nebeneinanderliegenden Windungen des Drahts (521) des lose gewickelten Abschnitts (522) angeordnet ist, und
der wenigstens eine aus dem distalen Endabschnitt (711, 721) und dem proximalen Endabschnitt (712, 722) des Markierungselements (71, 72) zur Innenschicht (51) hin gebogen ist.

2. Ballonkatheter (10) nach Anspruch 1, wobei wenigstens einer aus dem distalen Endabschnitt (711, 721) und dem proximalen Endabschnitt (712, 722) des Markierungselements (71, 72) eine glatte und durchgängige Verjüngung bildet.

3. Ballonkatheter (10) nach Anspruch 1 oder 2, wobei sowohl der distale Endabschnitt (711, 721) als auch der proximale Endabschnitt (712, 722) des Markierungselements (71, 72) zur Innenschicht (51) hin gebogen sind.

4. Ballonkatheter (10) nach Anspruch 3, wobei der wenigstens eine aus dem distalen Endabschnitt (711, 721) und dem proximalen Endabschnitt (712, 722) des Markierungselements (71, 72) sich vollständig in der Lücke (54) befindet.

5. Ballonkatheter (10) nach einem der Ansprüche 1 bis 4, wobei das Markierungselement (72) aus einem parallelogrammförmigen Metallblech (A) gebildet ist, das so um den Außenumfang des Wicklungskörpers (52) gewickelt ist, dass der distale Endabschnitt (721) und der proximale Endabschnitt (722) an einer Stelle gegenüber einer Lücke (54) angeordnet sind.

## Revendications

1. Cathéter à ballonnet (10) comprenant :
un ballonnet (20) ;
une tige extérieure (30) jointe au ballonnet (20) ; et
une tige intérieure (50) jointe au ballonnet (20), la tige intérieure (50) comprenant
une couche intérieure (51) ;
un corps de bobine (52) disposé sur la périphérie extérieure de la couche intérieure (51) et ayant une pluralité de tours d'un fil métallique (521), dans lequel le corps de bobine (52) présente une partie peu enroulée (522) où des espaces sont formés entre les tours adjacents du fil métallique (521) ;
un élément marqueur (70, 71, 72) disposé sur la périphérie extérieure du corps de bobine (52) ;
**caractérisé en ce que**
le cathéter à ballonnet (10) comprend en outre une couche extérieure (53) couvrant le corps de bobine (52) et l'élément marqueur (71, 72),
au moins l'une d'une partie extrémité distale (711, 721) et d'une partie extrémité proximale (712, 722) de l'élément marqueur (71, 72) est disposée au niveau d'une position faisant face à un vide (54) formé entre les tours adjacents du fil métallique (521) de la partie peu enroulée (522), et
l'au moins une de la partie extrémité distale (711, 721) et de la partie extrémité proximale (712, 722) de l'élément marqueur (71, 72) est incurvée en direction de la couche intérieure (51).

2. Cathéter à ballonnet (10) selon la revendication 1, dans lequel au moins l'une de la partie extrémité distale (711, 721) et de la partie extrémité proximale (712, 722) de l'élément marqueur (71, 72) forme une forme conique lisse et continue.

3. Cathéter à ballonnet (10) selon la revendication 1 ou 2, dans lequel à la fois la partie extrémité distale (711, 721) et la partie extrémité proximale (712, 722) de l'élément marqueur (71, 72) sont incurvées en direction de la couche intérieure (51).

4. Cathéter à ballonnet (10) selon la revendication 3, dans lequel l'au moins une de la partie extrémité distale (711, 721) et de la partie extrémité proximale (712, 722) de l'élément marqueur (71, 72) réside complètement à l'intérieur du vide (54).

5. Cathéter à ballonnet (10) selon l'une quelconque des revendications 1 à 4, dans lequel l'élément marqueur (72) est formé par une plaque métallique parallélogramme (A) enroulée sur la périphérie extérieure du corps de bobine (52) de manière à ce que la partie extrémité distale (721) et la partie extrémité proximale (722) soient disposées au niveau d'une position faisant face à un vide (54).
